# EUROPEAN PATENT SPECIFICATION

(11) **EP 3 781 932 B1**
(45) Date of publication and mention of the grant of the patent: **29.11.2023**
(21) Application number: 19724928.7
(22) Date of filing: 15.04.2019
(51) Int. Cl.: G01N 22/00, A61B 5/0507

(54) **DEVICE FOR MICROWAVE MEASUREMENT OF A DIELECTRIC DISCONTINUITY OF A MATERIAL**
VORRICHTUNG ZUR MIKROWELLENMESSUNG EINER DIELEKTRISCHEN DISKONTINUITÄT EINES MATERIALS
DISPOSITIF DE MESURE PAR MICRO-ONDES D'UNE DISCONTINUITÉ DIÉLECTRIQUE D'UN MATÉRIAU

(30) Priority: 17.04.2018 IT 201800004591; 10.07.2018 IT 201800007063
(43) Date of publication of application: 24.02.2021
(73) Proprietor: UBT S.R.L., 06081 Assisi (PG) (IT)
(72) Inventor: RASPA, Giovanni, 06080 Rivotorto Di Assisi (Perugia) (IT); TIBERI, Gianluigi, 06036 Montefalco (Perugia) (IT)
(74) Representative: Penza, Giancarlo
(86) International application number: PCT/IB2019/053074
(87) International publication number: WO 2019/202464

(56) References cited:
- WO-A1-2016/005909
- US-A- 4 274 048
- US-A1- 2009 027 288
- US-A1- 2015 230 725
- US-B1- 6 216 025
- PADHI SHANTANU ET AL: "A PC-controlled microwave tomographic scanner for breast imaging", REVIEW OF SCIENTIFIC INSTRUMENTS, AIP, MELVILLE, NY, US, vol. 82, no. 1, 21 January 2011 (2011-01-21), pages 14702-14702, XP012146106, ISSN: 0034-6748, DOI: 10.1063/1.3523048
- VLADIMIR MATVEJEV ET AL: "INTEGRATED WAVEGUIDE STRUCTURE FOR HIGHLY SENSITIVE THZ SPECTROSCOPY OF NANO-LITER LIQUIDS IN CAPILLARY TUBES", PROGRESS IN ELECTROMAGNETICS RESEARCH, vol. 121, 31 December 2011 (2011-12-31), pages 89-101, XP055157270, DOI: 10.2528/PIER11090102

## Description

### TECHNICAL FIELD OF THE INVENTION

The present invention relates to the technical field of electromagnetic detecting apparatus.

In particular, the present invention concerns a device for microwave measurement, especially suitable for controlling a dielectric homogeneity of objects or portions of human body.

The device further allows to obtain images representative of a map of said dielectric homogeneity.

### PRIOR ART

The control of dielectric homogeneity of objects represents, in the field of production activities, an aspect linked to quality control of semi-finished pieces and finished products.

In the medical field, control of the dielectric homogeneity is linked for example to the diagnosis of cancers, the classification of strokes and bone analysis.

The use of optical techniques is known with the aim of carrying out images processing of a material the properties of which are to be studied.

In the industrial field, these devices are directed at non-destructive control of production processes, with the aim of guaranteeing high quality of the finished products and, in the final analysis, ensuring that one or more production parameters are satisfied as a function of which the effective marketability of the analysed product is determined.

Still more important is the use of these techniques in the medical field, where they can be exploited for diagnostic purposes, in this way allowing the discovery of pathologies and disorders of the human body.

Therefore it is clear that the efficiency of the devices exploiting these techniques is of fundamental importance, in particular as regards the quality of the images gathered, so as to be able to diagnose in a timely and detailed manner the onset of any pathologies that are injurious to the health.

The known optical methods substantially provide topographical reconstructions of the material that is to be inspected using various means:
- ultrasound scanners;
- X-ray scanners (known as Computed Tomography (CT));
- analyses using nuclear magnetic resonance imaging (known as MRI).

However, all the above-indicated methodologies have significant drawbacks which make them very poorly efficient in use for diagnostic purposes, in particular from the point of view of the quality of the information gathered and the potential risks that the use of these methodologies might cause for a patient.

Over time the microwave imaging analysis has gradually attracted greater attention.

The microwave imaging analysis is effective each time there is a contrast (i.e. a difference) in dielectric constant and/or conductivity between portions of material that are to be analysed, whether this is a semi-finished piece in an industrial context or human tissue in the medical field.

In particular in the latter field, the microwave analysis can be substantially subdivided into two fields:
- microwave tomography;
- ultra wide band (UWB) radio techniques.

Unfortunately, in this case too there are intrinsic limitations which reduce the predictive capacity thereof.

Specifically, tomography is extremely complex in terms of modelling as it requires the solution of a non-linear inverse problem, and in UWB complex focusing techniques are necessary; in some cases filters are necessary to improve the suppression of possible disturbing images (clutter suppression) and spatial discrimination which, however, require an increase in system complexity.

Further, a substantial problem of known systems is the need to implement an anechoic chamber and/or a matching liquid located around the measurement device, which allows to increase resolution by means of a reduction of interference and noise.

An example of these devices is shown in document US 6454711 B1 which discloses a microwave device for analysis of the haemorrhagic stroke; the device uses an array constituted by numerous antennae (#1, #2, ... #16,) and is composed of a helmet which requires the use of an adapting liquid.

WO 2014/045181 discloses an apparatus for controlling the integrity of a mammary tissue by means of microwave imaging analysis. US6216025 is another document disclosing an apparatus for performing microwave imaging. However, the microwave radiation reflected from the imaged material is not conveyed to a receiving antenna by means of a waveguide.

US 2009/027288 discloses an antenna device for diagnosing cancer using microwave imaging. The antenna device includes a tank, an antenna frame and an array antenna. The tank has an opening and includes a medium having a relative dielectric constant that corresponds to the tissue of the target. The antenna frame is formed in the tank. The array antenna is mounted to the antenna frame and includes a plurality of antennas that vertically extend so as to surround the target that is inserted through the opening. A first antenna of the antennas includes a transmitting module that transmits microwave signals and other second antennas include receiving modules that receive the transmitted microwave signals.

Padhi Shantanu et al., "A PC-controlled microwave tomographic scanner for breast imaging", Review of Scientific Instruments, AlP, Melville, NY, US, vol.82, no. 1, 21 January 2011, pages 14702-14702, discloses a PC-based controller for a microwave tomographic system for breast cancer detection.

WO 2016/005909 discloses an imaging setup including a plurality of antennas assemblies positioned (completely or partially) around an object of interest, which is located in a measurement chamber.

### SUMMARY OF THE INVENTION

In this context, the technical task underlying the present invention is to provide a microwave measurement device which obviates at least some of the drawbacks in the prior art as described above.

In particular, an aim of the present invention is to provide a device for microwave measurement of at least a dielectric discontinuity of a material to be inspected, such as for example objects or portions of human body such as the breast, limbs and the head.

A further aim is to provide a measurement device which allows both determining a presence of an inhomogeneity within a material to be inspected (in particular, a dielectric non-homogeneity) and precisely determining its position inside the material.

The device further allows to obtain an image representing a map of dielectric homogeneity of the inspected material.

The technical task set and the objects specified are substantially achieved by a microwave measurement device, comprising the technical features as set out in one or more of the accompanying claims.

According to the present invention, a device is described for microwave measurement of at least a dielectric discontinuity of a material to be inspected, the device comprising a launcher for a waveguide, a waveguide and a receiving antenna. The launcher is configured to emit in the waveguide a microwave electromagnetic field adapted to interact with the material to be inspected. The receiving antenna is configured to receive an electromagnetic field reflected by the material to be inspected, said reflected electromagnetic field containing information correlated with a dielectric discontinuity of said material.

The waveguide is configured to propagate the electromagnetic field emitted towards the material to be inspected and to convey the reflected electromagnetic field towards the receiving antenna.

The waveguide has an inspection seat configured for the insertion of at least one portion of the material to be inspected, wherein the inspection seat is a hole in a wall of the waveguide.

At least one of either the launcher or the receiving antenna is movable (for example in rotation) in a plurality of positions around the inspection seat.

The dependent claims herein incorporated for reference, correspond to different embodiments of the invention.

### BRIEF DESCRIPTION OF THE DRAWINGS

Further characteristics and advantages of the present invention will become more apparent from the indicative and thus non-limiting description of a preferred but non-exclusive embodiment of a measurement device, as illustrated in the attached drawings, in which:

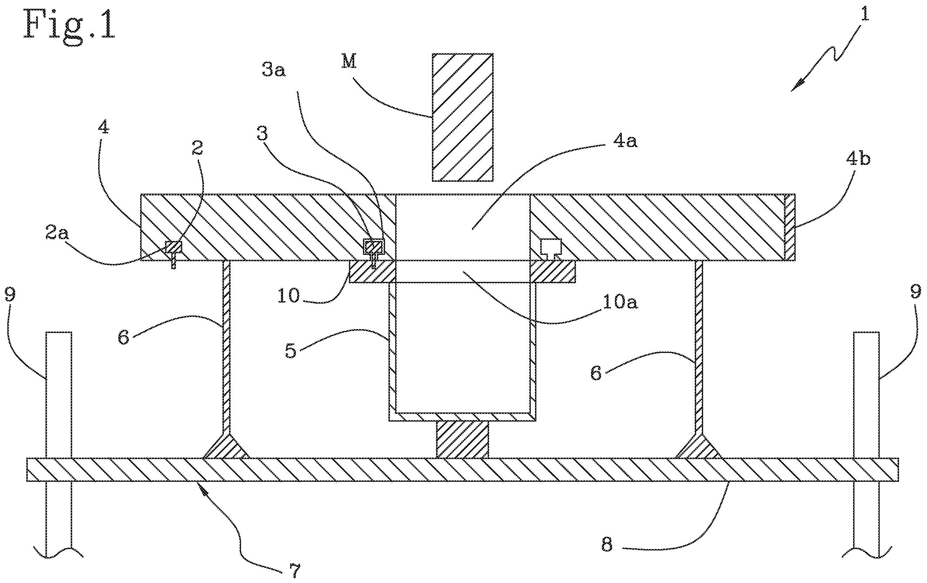

- figure 1 schematically shows a section view of a device for microwave measurement of a dielectric discontinuity of a material according to the present invention;

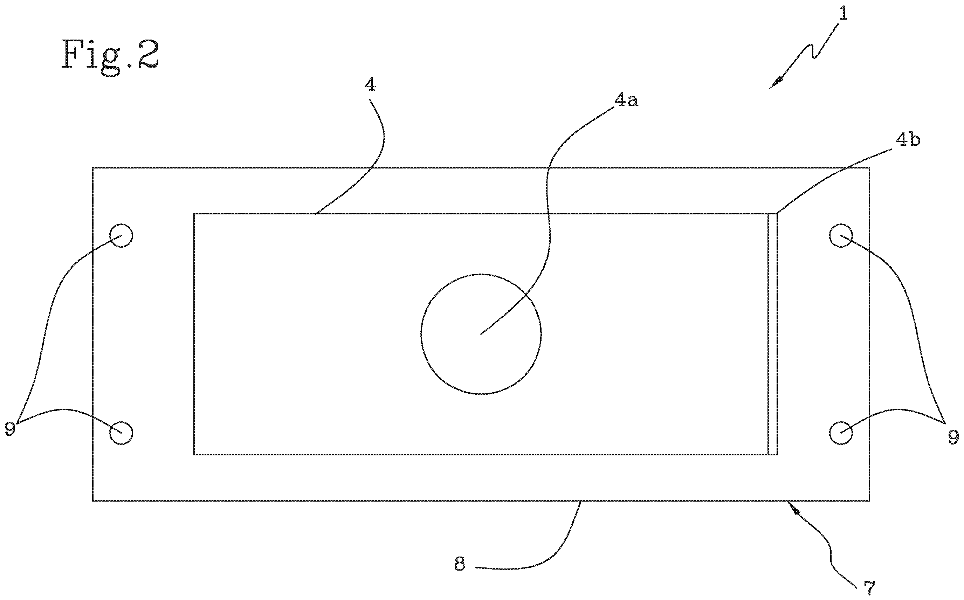

- figure 2 schematically shows a top view of some main components of the microwave measurement device of figure 1.

### DETAILED DESCRIPTION OF THE INVENTION

In the enclosed figures, reference numeral 1 denotes in its entirety a device for microwave measurement of at least a dielectric discontinuity of a material "M" to be inspected according to the invention.

The term microwave means a frequency band comprised between the minimum value of 0.3 Ghz and the maximum value of 300 Ghz.

The measurement device 1 comprises a waveguide 4, a launcher for waveguide 2 and a receiving antenna 3.

The launcher for waveguide 2 is configured to emit in the waveguide 4 an electromagnetic field in the microwave band adapted to interact with the material M to be inspected.

The launcher 2 is a source of a microwave electromagnetic field, which is for example generated by means of a measuring instrument denoted as "Vector Network Analyzer" or equivalent measuring instruments.

The waveguide 4 is configured to propagate the microwave electromagnetic field emitted by the launcher 2 over one or more modes towards the material "M" to be inspected and to convey towards the receiving antenna 3 the electromagnetic field reflected by the material M.

The receiving antenna 3 is configured to receive an electromagnetic field reflected by the material "M" to be inspected.

The waveguide 4 is for example of a type having a rectangular section with dimensions of the rectangular section being equal to 30 cm and 10 cm and with a length equal to about 80 cm.

Alternatively, the waveguide 4 can have a circular, elliptical or other-shaped section, appropriate for inspecting the material M.

During the operation of the measurement device 1, the waveguide 4 propagates the microwave electromagnetic field emitted by the launcher 2 over one or more modes towards the material "M" to be inspected.

The receiving antenna 3 thus receives the microwave electromagnetic field reflected by the material "M" containing information correlated to the dielectric discontinuity of the material "M" to be inspected.

The waveguide 4 thus has the function of propagating the microwave electromagnetic field emitted by the launcher 2 into one or more modes towards the material "M" to be inspected, in this way guaranteeing optimal focusing and concentration of the electromagnetic field, which is translated into an intensity of the reflected electromagnetic field that is greater by about 30 dB with respect to the known solutions.

Moreover, the waveguide 4 has the function of illuminating the material "M" to be inspected with the microwave electromagnetic field which has propagated into the waveguide, by means of the inspection seat 4a inside which the illuminated material "M" is inserted.

According to a preferred embodiment, the measurement device 1 comprises two launchers 2 configured to generate respective electromagnetic fields shifted each other by 90 degrees, so as to generate an overall electromagnetic field having a circular or elliptical polarisation state, provided that the waveguide 4 has appropriate dimensions and a geometrical shape: in this case the measurement device 1 comprises an appropriate pair of receiving antennas 3 configured to receive the overall electromagnetic field reflected into the two transversal components.

The waveguide 4 has an inspection seat 4a configured for the insertion of at least one portion of the material "M" to be inspected and the receiving antenna 3 is further movable in a plurality of positions around the inspection seat 4a.

According to the present invention, the inspection seat 4a is a hole in a wall of the waveguide 4, having an appropriate dimension to allow insertion of at least one portion of the material M inside the hole.

For example, the hole of the waveguide 4 has a substantially circular shape with a radius uqual for example to 6 centimetres, so as toallow for example the insertion of a woman's breast into the hole.

In particular, the receiving antenna 3 is movable in rotation (for example with a radius of 7 cm) around the inspection seat 4a, for example into 80 positions.

The inspection seat 4a (in particular the hole) is for example arranged in a central portion of the waveguide 4.

This means that, in a use configuration, the material "M" to be inspected is thus at least partly inserted inside the inspection seat 4a while the waveguide 4 and the launcher 2 and the receiving antenna 3 are at a certain height with respect to the material "M".

In this situation the receiving antenna 3 can be moved (for example, in rotation) around the inspection seat 4a (and thus around the portion of material M to be inspected which is internal thereof) with the aim of detecting the electromagnetic field reflected by the material M into a plurality of positions that are radially arranged around the material "M", thus optimizing the quantity and quality of the gathered information.

The material "M" might also be only partly inserted into the inspection seat 4a and consequently the information gathered by the measurement device 1 will be taken from the electromagnetic field reflected by the portion of material "M" which is at a certain time inside the inspection seat 4a.

In particular, the waveguide 4 is matched and it comprises (in at least a portion of at least an external edge thereof) a coating 4b made of an absorbing material adapted to absorb the electromagnetic fields in the used frequency band, in particular in the microwave band.

In other words, the waveguide 4 has absorbing coatings 4b arranged peripherally which allow to significantly reduce (even eliminate) the reflection of electromagnetic fields, this, from the electromagnetic point of view, prefigures the waveguide 4 as a guide having an infinite length.

Advantageously, the launcher 2 is such to emit in the waveguide 4 an electromagnetic field which will propagate both over the fundamental mode and over one or more higher order propagation modes, thus the waveguide 4 is a multimode waveguide which allows to exploit a wide frequency band within the microwave regime, in order to carry out the measuring process.
In fact, the highest frequencies (for example those in proximity of the upper limit of the operating band of the device) guarantee a high resolution of the information that can be acquired by means of the reflected electromagnetic field, while the lowest frequencies (for example those in proximity of the lower limit of the operating band of the device) guarantee excellent penetration of the material "M", thus allowing to obtain detailed and precise information also from the most internal portions thereof.
according to a first embodiment, shown in detail in the enclosed figures, the launcher 2 is arranged in a more radially external position, with respect to the material "M" to be inspected, of the receiving antenna 3.

In practice, the launcher 2 is positioned at a greater distance from the material "M" to be inspected, with respect to the receiving antenna 3.

Preferably, the receiving antenna is in close proximity with or adjacent to the material "M" to be inspected.

In a further possible embodiment, not shown in the enclosed figures, the positions of the launcher 2 and of the receiving antenna 3 are exchanged, i.e. the receiving antenna 3 is positioned at a greater distance from the material "M" to be inspected, with respect to the launcher 2.

The precise distance and positioning of the launcher 2 and of the receiving antenna 3 with respect to the inspection seat 4a are calculated and defined as a function of the specific working frequencies of the measurement device 1 and of the geometrical characteristics of the waveguide 4, so as to optimise the quality of the signal received at the receiving antenna 3.

Advantageously, the waveguide 4 has at least one external seat 2a configured to receive by insertion one out of the launcher 2 and the receiving antenna 3, this external seat 2a being arranged in a peripheral portion of the waveguide.

In other words, the waveguide 4 has an external seat 2a inside which the launcher 2 or the receiving antenna 3 can be inserted, in such a way that the electromagnetic field is generated or received directly inside the waveguide 4.

The waveguide 4 has an internal seat 3a for insertion of the launcher 2 or the receiving antenna 3, the internal seat 3a is arranged between the external seat 2a and the inspection seat 4a.

In other words, the external seat 2a is arranged in a more radially external position, with respect to the material M to be inspected, with respect to the internal seat 3a.

It follows that in accordance with the first embodiment outlined above, the launcher 2 is inserted in the external seat 2a, while the receiving antenna 3 is inserted in the internal seat 3a.

According to the second embodiment outlined above, the launcher 2 is instead inserted in the internal seat 3a, while the receiving antenna 3 is inserted in the external seat 2a.

In the hypothesis in which the material M to be inspected has a substantially circular geometrical conformation, in order to allow the movement in rotation around the inspection seat 4a of the launcher 2 or of the receiving antenna 3, depending on which is inserted inside it, the internal seat 3a has a circumferential extension around the inspection seat 4a substantially defining a ring structure, inside which the launcher 2 or the receiving antenna 3 can slide in rotation around the inspection seat 4a.

The inspection seat 4 is thus surrounded by the internal seat 3a in such a way that the launcher 2 or receiving antenna 3, sliding inside it, can assume a plurality of angular positions around the material "M" to be inspected, in this way generating or receiving the electromagnetic field or the electromagnetic field reflected from a plurality of directions: in this way the material "M" (positioned inside the hole 4a) is illuminated by the microwave electromagnetic field in various angular positions around the material "M".

More in general, the material to be inspected can have a generic geometrical conformation: in this case the launcher 2 or the receiving antenna 3 is adapted to follow a perimeter path around the material M to be inspected, for example by defining an interpolated perimeter path (of the launcher 2 or the receiving antenna 3) identified by a pair of vectors obtainable by means of two motors or equivalent means.

The apparatus 1 further comprises a support element 10, preferably arranged below the waveguide, which has a seat 10a coaxial with said inspection seat 4a.

The receiving antenna 3 is constrained to the support element 10, preferably in a position adjacent to the seat 10a.

In order to allow the detection of the reflected electromagnetic field from a plurality of positions around the material "M" to be inspected, the measurement device 1 thus comprises first rotating means 5 associated with the support element 10 and configured to move the support element 10 in rotation around the inspection seat 4a, consequently determining a corresponding rotation of the receiving antenna 3 around the inspection seat 4a.

In the same way the external seat 2a can also be moved with respect to the material M to be inspected, so as to cause the launcher 2 to assume various positions and thus strike the material "M" with an electromagnetic field coming from different directions.

In accordance with this aspect, the measurement device 1 further comprises second rotation means 6 configured to actuate a relative rotational motion between the waveguide 4 and the material "M".

According to a preferred embodiment, the second rotation means are associated with the waveguide 4 and configured to move the waveguide 4 in rotation in a plurality of positions (for example 15) around a geometric centre of the inspection seat 4a.

In other words, the measurement device 1 of the present invention can comprise rotating means 5, 6 configured to set the support element 10 and/or the whole waveguide 4 in rotation around the material M to be inspected. The launcher 2 or the receiving antenna 3 are associated to the support element 10 and/or the whole waveguide 4 in such a way that at least one of the two is movable in rotation around the inspection seat 4a.

This special aspect of the embodiment allows moving the launcher 2 and the receiving antenna 3 for the generation and receiving of the electromagnetic field in such a way as to allow the acquisition of information around to the material "M" to be inspected.

Preferably, the second rotation means 6 are configured to rotate the waveguide 4 around the inspection seat 4a, for example into 15 positions, thus for each thereof the first rotating means 5 are configured to rotate the receiving antenna 3, for example into 80 positions, thus obtaining a total of 15 x 80 = 1200 different positions around the inspection seat 4a (and thus around the material M to be inspected).

In particular, the second rotation means 6 can be made using a swivel ring coupled to the waveguide 4, preferably below the waveguide 4, and moved in rotation by appropriate motor means.

In order to ensure correct maintaining of the contacts necessary for the electrical supply of the measurement device 1 during the various rotational movements to which the different constituting elements of the measurement device 1 can be subjected, two rotating joints are present, coaxial to the inspection seat 4a and to which the cabling necessary for the correct operation of the measurement device 1 is connected.

The rotating joints have a fixed casing, associable to a rest base of the measurement device 1, and a movable internal member connected by an arm to at least one of the rotating means 5, 6, in such a way as to be set thereby in rotation solidly with the waveguide 4 and/or the support element 10.

Alternatively, the second rotating means 6 are associable to the material "M" or to a containment element of the material "M" and are configured to move the material "M" in rotation in a plurality of positions (for example 15) around a geometric centre of the inspection seat 4a.

The measurement device 1 further comprises a movement member 7 associated to the waveguide 4 and configured to vary the height in which the waveguide 4 is located with respect to the material "M" to be inspected, determining a greater or lesser insertion inside it.

Alternatively, the measurement device 1 comprises a containment element of the material "M" and the movement member 7 is associated to this containment element in order to vary the height in which the material "M" is located with respect to the waveguide 4, determining a greater or lesser insertion inside it.

Alternatively, the movement member is directly couplable to the material "M" with the purpose of moving the material "M" to determine the height thereof with respect to the waveguide 4.

This specification allows to vary the relative position between the material "M" and the waveguide 4, as it modifies the portion of material "M" which is struck by the electromagnetic field and which consequently will generate the reflected electromagnetic field by means of which important information relating to the characteristics of the material "M" can be obtained.

In accordance with a special embodiment, the movement member 7 can be made using a moveable frame which comprises a support plane 8 that is substantially horizontal, on which the launcher 2 and the receiving antenna 3 and the waveguide 4 and the relative rotating means 5, 6 (if present) and a hub 9 are installed, passing through the support plane 8 and along which this plane, which performs the guide function, is slidable.

In order to allow the movement of the support plane 8, and thus of the elements arranged thereon, the movement member 7 can comprise a ball screw on which the support plane 8 slides, determining a movement in a substantially vertical or horizontal direction where the application requires it.

Alternatively, the vertical movement of the support plane 8 can be implemented using equivalent mechanical means such as belts, racks, chains, or other known solutions.

According to a particular aspect of the present invention, the measurement device 1 is a device for controlling the integrity of the biological tissues in which the material "M" to be inspected comprises a biological tissue, such as, by way of non-limiting example, a mammary tissue, a head, a limb; for this reason the inspection seat 4a is adapted to contain this biological tissue.

Again according to this special aspect, the dielectric discontinuities that are to be detected by means of the measurement device 1 are an inhomogeneity of the biological tissue, in particular a significant variation (for example an increase by at least 20%) of the dielectric constant and/or the electrical conductivity of the biological tissue.

The measurement device 1 is thus configured to determine the presence of a non-integrity inside the biological tissue, on the ground of the determination on any eventual discontinuities in the dielectric behaviour of the biological tissue.

The term "non-integrity" means a dishomogeneity in the biological tissue indicating anomalous conditions of composition of the tissue.

In practice, the present invention allows to identify, within the mass of biological tissue (supposed to be homogeneous, within some limits), zones in which the relative dielectric constant and/or the conductivity have significant variations (for example an increase of at least 20%).

In this particular context the following operating situation is described, introduced purely by way of non-limiting example, with the aim of better delineating some special aspects of the present invention.

Let's consider a biological tissue illuminated by an electromagnetic field in the microwave frequency band and which propagates inside the waveguide 4, in which the illuminated biological tissue is positioned inside the inspection seat 4a of the waveguide 4 (i.e. inside a hole 4a in the waveguide 4 in accordance with the present invention).

It is further supposed that if the biological tissue has homogeneity of behaviour with respect to the dielectric constant and the conductivity, then it can be considered integral, i.e. free of detectable pathologies.

Viceversa, in case wherein dielectric discontinuities were to be revealed, then the assessment of integrity would change and there might be cases of issues in the material.

According to an embodiment of the invention, a map of dielectric homogeneity is calculated within the biological tissue as a function of the reflected electromagnetic field detected by the receiving antenna 3 and exploiting the Huygens principle.

If the biological tissue contains a non-integrity, the map of dielectric homogeneity will show a signal peak at the position of the non-integrity.

The map of dielectric homogeneity inside the biological tissue is calculated inside as a function of the reflected electromagnetic field detected by the receiving antenna 3 and exploiting the Huygens principle: this calculation of the electromagnetic field inside the mammary tissue by means of the Huygens principle is explained in detail in the document by Navid Ghavami, Gianluigi Tiberi, David J. Edwards, Agostino Monorchio, "UWB Microwave Imaging of Objects With Canonical Shape", IEEE Transactions on antennas and propagation, IEEE service centerm Piscataway, NJ, US, vol.60, no.1, January 1st 2012, pages 231-239.

With the aim of eliminating any artefacts, a succession of measurements can be made, which are subdivided into measurement groups carried out in adjacent positions, processing the signals thus obtained and performing a difference, the quality of the microwave image (i.e. the map of dielectric homogeneity) obtainable by the use of the measurement device 1 is much improved.

With the aim of carrying out a processing of three-dimensional images , the described and claimed process can be repeated various times, by positioning the waveguide 4 at different heights along the height (i.e. the longitudinal extension) of the biological tissue, in this way gathering a series of a plurality of data, each of which contains a plurality of values of the electromagnetic field reflected from various points of the biological tissue, obtained at a particular height.

The relative movement between the waveguide 4 and the material "M" can be obtained either by moving the guide 4 away from or near to the material "M", which is instead kept fixed, or by moving the material "M", determining a greater or lesser insertion inside the inspection seat 4a of the guide 4 which is instead kept fixed.

In particular, the first above-mentioned case is preferable in a case in which the material "M" is a biological tissue and the inspection has a diagnostic purpose, with the aim of reducing to a minimum the disturbance to the patient deriving from the diagnostic procedure.

The present invention further relates to a microwave measurement apparatus comprising a measurement device 1.

In particular, the microwave measurement apparatus includes the measurement device 1 comprising one or more of the technical features described above, and comprises a processing unit and a Vector Network Analyzer or an equivalent measuring instrument.

The Vector Network Analyzer comprises at least an output port connected to the launcher 2 and it is such to emit the microwave electromagnetic field inside the waveguide 4.

The Vector Network Analyzer comprises at least an input port connected to the receiving antenna 3 and it is such to detect the amplitude and phase of the reflected electromagnetic field received by the receiving antenna 3; said reflected electromagnetic field is generally denoted by "S21".

The processing unit is configured to receive a plurality of values (for example 1601) representing the amplitude and phase of the electromagnetic field reflected by the material M to be inspected, it is configured to process said plurality of values of the amplitude and phase of the reflected electromagnetic field, it is configured to generate, as a function of said processing, an image representative of the map of dielectric homogeneity of the material M and a signal representative of the presence or absence of a non-integrity of the material M.

Preferably, the processing unit is configured to generate, as a function of said plurality of values of the amplitude and phase of the electromagnetic field reflected by the material M, a microwave image representing a map of dielectric homogeneity of the material M.

Preferably, in case of detection of presence of a non-integrity of the material M, the processing unit is further configured to generate a microwave image representative of the position of the non-integrity inside the material M.

The processing unit can be a microprocessor or a similar architecture.

The present invention advantageously attains the proposed objectives and obviates the perceived drawbacks in the known art by providing a measurement device 1 which allows measuring, in a non- invasive way which is at the same time reliable, a dielectric discontinuity of a material "M".

The measurement device 1 further allows an optimal positioning of the launcher 2 and of the receiving antenna 3 in order to guarantee optimal control of the material M.

Advantageously, the presence of the waveguide 4, and in particular the inspection seat 4a inside which the material "M" to be inspected is arranged, allows to optimize the quality of the used signals, thus significantly improving the efficiency of the measurement device 1 itself.

Further, the presence of the waveguide 4 allows to implement a compact system, in air, and efficiently miniaturised, thus obviating the need to use a large anechoic chamber for optimisation of the collecting of the signals used for the measuring process.

## Claims

1. Device for microwave measurement of a dielectric discontinuity of a material (M) to be inspected, said device (1) comprising a launcher for waveguide (2), a waveguide (4) and a receiving antenna (3), wherein:
- the launcher (2) is configured to emit in the waveguide a microwave electromagnetic field adapted to interact with the material (M) to be inspected;
- the receiving antenna (3) is configured to receive an electromagnetic field reflected by the material (M), said reflected electromagnetic field containing information correlated with a dielectric discontinuity of said material (M);
- the waveguide (4) is configured to:
• propagate the emitted electromagnetic field towards the material (M);
• convey the reflected electromagnetic field towards the receiving antenna (3);
said waveguide (4) having an inspection seat (4a) configured for the insertion of at least one portion of the material (M), wherein the inspection seat (4a) is a hole in a wall of the waveguide (4),
and wherein at least one of either the launcher (2) or the receiving antenna (3) is movable in a plurality of positions around the inspection seat (4a).

2. Device according to claim 1, wherein the receiving antenna (3) is movable in rotation in a plurality of positions around the inspection seat (4a).

3. Device according claims 1 or 2, wherein the inspection seat (4a) is arranged in a central portion of the waveguide (4).

4. Device according to any one of claims 1 to 3, wherein the waveguide (4) has an external seat (2a) for insertion of the launcher (2) and an internal seat (3a) for insertion of the receiving antenna (3), wherein the external seat (2a) is arranged in a more radially external position with respect to the internal seat (3a),
wherein the external seat (2a) is arranged at a peripheral portion of the waveguide (4) and wherein the internal seat (3a) is arranged between the external seat (2a) and the inspection seat (4a).

5. Device according to any one of claims 1 to 3, wherein the waveguide (4) has an external seat (2a) for insertion of the receiving antenna and an internal seat (3a) for insertion of the launcher, wherein the external seat (2a) is arranged in a more radially external position with respect to the internal seat (3a),
wherein the external seat (2a) is arranged at a peripheral portion of the waveguide (4) and wherein the internal seat (3a) is arranged between the external seat (2a) and the inspection seat (4a).

6. Device according to claim 4 or 5, wherein the internal seat (3a) has a circumferential extension around the inspection seat (4a) and the launcher (2) or the receiving antenna (3) is slidable, inside the internal seat (3a), in rotation around the inspection seat (4a).

7. Device according to any one of the preceding claims, further comprising a support element (10) arranged below the waveguide (4) and having a seat (1 0a) coaxial with said inspection seat (4b), the launcher (2) or the receiving antenna (3) being constrained to said support element (10), the device further comprising first rotation means (5) associated with said support element (10) and configured to move said support element (10) in rotation, determining a corresponding rotation of the launcher (2) or of the receiving antenna (3) in a plurality of positions around the inspection seat (4a).

8. Device according to any one of the preceding claims, comprising second rotation means (6) associated with the waveguide (4) and configured to move said waveguide (4) in rotation in a plurality of positions around a geometric centre of the inspection seat (4a).

9. Device according to any one of preceding claims 1-8, comprising second rotation means (6) associated with the material (M) to be inspected and configured to move said material (M) in rotation in a plurality of positions coaxially with respect to a geometric centre of the inspection seat (4a).

10. Device according to any one of the preceding claims, comprising a movement member (7) configured to change a relative distance between the waveguide (4) and the material (M) to be inspected, determining a greater or lesser insertion of the material (M) to be inspected inside the inspection seat (4).

11. Device according to any one of the preceding claims, wherein the waveguide (4) comprises, in at least one portion of at least one outer edge of the waveguide (4), a coating made of an absorbing material (M) adapted to at least partially absorb the electromagnetic field emitted in the waveguide.

12. Device according to any one of the preceding claims, wherein the waveguide (4) is configured to propagate the electromagnetic field of a plurality of modes of propagation towards the material.

13. Microwave measurement apparatus comprising:
- a measurement device (1) according to one or more of the preceding claims;
- a processing unit configured to:
• process a plurality of values indicating amplitude and phase of the electromagnetic field reflected by the material in a plurality of different relative positions between the material and the receiving antenna;
• generate, as a function of said processing, an image representative of a map of dielectric homogeneity of the material and a signal representative of the presence or absence of a non-integrity of the material.

## Patentansprüche

1. Vorrichtung zur Mikrowellenmessung einer dielektrischen Diskontinuität eines zu inspizierenden Materials (M), wobei die Vorrichtung (1) einen Wellenleiterwerfer (2), einen Wellenleiter (4) und eine Empfangsantenne (3) umfasst, wobei:
- der Werfer (2) ausgelegt ist, um in dem Wellenleiter ein elektromagnetisches Mikrowellenfeld auszusenden, das geeignet ist, mit dem zu inspizierenden Material (M) zusammenzuwirken,
- die Empfangsantenne (3) ausgelegt ist, um ein elektromagnetisches Feld zu empfangen, das von dem Material (M) reflektiert wird, wobei das reflektierte elektromagnetische Feld Informationen enthält, die mit einer dielektrischen Diskontinuität des Materials (M) korreliert sind,
- der Wellenleiter (4) ausgelegt ist, um:
• das ausgesendete elektromagnetische Feld in Richtung des Materials (M) auszubreiten,
• das reflektierte elektromagnetische Feld zur Empfangsantenne (3) weiterzuleiten,
wobei der Wellenleiter (4) einen Inspektionssitz (4a) aufweist, der zum Einfügen von mindestens einem Abschnitt des Materials (M) ausgelegt ist, wobei der Inspektionssitz (4a) ein Loch in einer Wand des Wellenleiters (4) ist,
und wobei mindestens entweder der Werfer (2) oder die Empfangsantenne (3) in mehreren Positionen rund um den Inspektionssitz (4a) bewegbar ist.

2. Vorrichtung nach Anspruch 1, wobei die Empfangsantenne (3) in mehreren Positionen in Rotation um den Inspektionssitz (4a) bewegbar ist.

3. Vorrichtung nach Anspruch 1 oder 2, wobei der Inspektionssitz (4a) in einem zentralen Abschnitt des Wellenleiters (4) angeordnet ist.

4. Vorrichtung nach einem der Ansprüche 1 bis 3, wobei der Wellenleiter (4) einen Außensitz (2a) zum Einfügen des Werfers (2) und einen Innensitz (3a) zum Einfügen der Empfangsantenne (3) aufweist, wobei der Außensitz (2a) in Bezug auf den Innensitz (3a) in einer radial weiter außen liegenden Position angeordnet ist,
wobei der Außensitz (2a) an einem Umfangsabschnitt des Wellenleiters (4) angeordnet ist und wobei der Innensitz (3a) zwischen dem Außensitz (2a) und dem Inspektionssitz (4a) angeordnet ist.

5. Vorrichtung nach einem der Ansprüche 1 bis 3, wobei der Wellenleiter (4) einen Außensitz (2a) zum Einfügen der Empfangsantenne und einen Innensitz (3a) zum Einfügen des Werfers aufweist, wobei der Außensitz (2a) in Bezug auf den Innensitz (3a) in einer radial weiter außen liegenden Position angeordnet ist,
wobei der Außensitz (2a) an einem Umfangsabschnitt des Wellenleiters (4) angeordnet ist und wobei der Innensitz (3a) zwischen dem Außensitz (2a) und dem Inspektionssitz (4a) angeordnet ist.

6. Vorrichtung nach Anspruch 4 oder 5, wobei der Innensitz (3a) eine Umfangserstreckung um den Inspektionssitz (4a) aufweist und der Werfer (2) oder die Empfangsantenne (3) im Inneren des Innensitzes (3a) in Rotation um den Inspektionssitz (4a) verschiebbar ist.

7. Vorrichtung nach einem der vorhergehenden Ansprüche, ferner umfassend ein Trägerelement (10), das unterhalb des Wellenleiters (4) angeordnet ist und einen Sitz (10a) aufweist, der koaxial zum Inspektionssitz (4b) ist, wobei der Werfer (2) oder die Empfangsantenne (3) an dem Trägerelement (10) befestigt ist, wobei die Vorrichtung ferner erste Rotationsmittel (5) umfasst, die mit dem Trägerelement (10) verbunden sind und ausgelegt sind, um das Trägerelement (10) in Rotation zu bewegen, wodurch eine entsprechende Rotation des Werfers (2) oder der Empfangsantenne (3) in mehreren Positionen um den Inspektionssitz (4a) bestimmt wird.

8. Vorrichtung nach einem der vorhergehenden Ansprüche, umfassend zweite Rotationsmittel (6), die mit dem Wellenleiter (4) verbunden sind und ausgelegt sind, um den Wellenleiter (4) in mehreren Positionen um eine geometrische Mitte des Inspektionssitzes (4a) in Rotation zu bewegen.

9. Vorrichtung nach einem der vorhergehenden Ansprüche 1-8, umfassend zweite Rotationsmittel (6), die mit dem zu inspizierenden Material (M) verbunden sind und ausgelegt sind, um das Material (M) in mehreren Positionen koaxial zu einer geometrischen Mitte des Inspektionssitzes (4a) in Rotation zu bewegen.

10. Vorrichtung nach einem der vorhergehenden Ansprüche, umfassend ein Bewegungsglied (7), das ausgelegt ist, um einen relativen Abstand zwischen dem Wellenleiter (4) und dem zu inspizierenden Material (M) zu ändern, wodurch eine größere oder geringere Einfügung des zu inspizierenden Materials (M) in den Inspektionssitz (4) bestimmt wird.

11. Vorrichtung nach einem der vorhergehenden Ansprüche, wobei der Wellenleiter (4) in mindestens einem Abschnitt von mindestens einer Außenkante des Wellenleiters (4) eine Beschichtung umfasst, die aus einem absorbierenden Material (M) besteht, das geeignet ist, das elektromagnetische Feld, das im Wellenleiter ausgesendet wird, mindestens teilweise zu absorbieren.

12. Vorrichtung nach einem der vorhergehenden Ansprüche, wobei der Wellenleiter (4) ausgelegt ist, um das elektromagnetische Feld mehrerer Ausbreitungsmodi in Richtung des Materials auszubreiten.

13. Mikrowellen-Messgerät, umfassend:
- eine Messvorrichtung (1) nach einem oder mehreren der vorhergehenden Ansprüche,
- eine Verarbeitungseinheit, die ausgelegt ist, um:
• mehrere Werte zu verarbeiten, die die Amplitude und Phase des elektromagnetischen Feldes angeben, das von dem Material in mehreren verschiedenen relativen Positionen zwischen dem Material und der Empfangsantenne reflektiert wird,
• in Abhängigkeit von dem Verarbeiten ein Bild, das für eine Karte der dielektrischen Homogenität des Materials repräsentativ ist, und ein Signal zu erzeugen, das für die Anwesenheit oder Abwesenheit einer mangelnden Unversehrtheit des Materials repräsentativ ist.

## Revendications

1. Dispositif de mesure par micro-ondes d'une discontinuité diélectrique d'un matériau (M) à inspecter, ledit dispositif (1) comprenant un lanceur pour guide d'ondes (2), un guide d'ondes (4) et une antenne de réception (3), dans lequel :
- le lanceur (2) est configuré pour émettre dans le guide d'ondes un champ électromagnétique micro-ondes adapté pour interagir avec le matériau (M) à inspecter ;
- l'antenne de réception (3) est configurée pour recevoir un champ électromagnétique réfléchi par le matériau (M), ledit champ électromagnétique réfléchi contenant des informations corrélées à une discontinuité diélectrique dudit matériau (M) ;
- le guide d'ondes (4) est configuré pour :
• propager le champ électromagnétique émis vers le matériau (M) ;
• acheminer le champ électromagnétique réfléchi vers l'antenne de réception (3) ;
ledit guide d'ondes (4) ayant un siège d'inspection (4a) configuré pour l'insertion d'au moins une portion du matériau (M), dans lequel le siège d'inspection (4a) est un trou dans une paroi du guide d'ondes (4),
et dans lequel au moins l'un du lanceur (2) ou de l'antenne de réception (3) est mobile dans une pluralité de positions autour du siège d'inspection (4a).

2. Dispositif selon la revendication 1, dans lequel l'antenne de réception (3) est mobile en rotation dans une pluralité de positions autour du siège d'inspection (4a).

3. Dispositif selon la revendication 1 ou 2, dans lequel le siège d'inspection (4a) est agencé dans une portion centrale du guide d'ondes (4).

4. Dispositif selon l'une quelconque des revendications 1 à 3, dans lequel le guide d'ondes (4) a un siège externe (2a) pour l'insertion du lanceur (2) et un siège interne (3a) pour l'insertion de l'antenne de réception (3), dans lequel le siège externe (2a) est agencé dans une position plus radialement externe par rapport au siège interne (3a),
dans lequel le siège externe (2a) est agencé au niveau d'une portion périphérique du guide d'ondes (4) et dans lequel le siège interne (3a) est agencé entre le siège externe (2a) et le siège d'inspection (4a).

5. Dispositif selon l'une quelconque des revendications 1 à 3, dans lequel le guide d'ondes (4) a un siège externe (2a) pour l'insertion de l'antenne de réception et un siège interne (3a) pour l'insertion du lanceur, dans lequel le siège externe (2a) est agencé dans une position plus radialement externe par rapport au siège interne (3a),
dans lequel le siège externe (2a) est agencé au niveau d'une portion périphérique du guide d'ondes (4) et dans lequel le siège interne (3a) est agencé entre le siège (2a) et le siège d'inspection (4a).

6. Dispositif selon la revendication 4 ou 5, dans lequel le siège interne (3a) a une extension circonférentielle autour du siège d'inspection (4a) et le lanceur (2) ou l'antenne de réception (3) peut coulisser, à l'intérieur du siège interne (3a), en rotation autour du siège d'inspection (4a).

7. Dispositif selon l'une quelconque des revendications précédentes, comprenant en outre un élément de support (10) agencé sous le guide d'ondes (4) et ayant un siège (10a) coaxial audit siège d'inspection (4b), le lanceur (2) ou l'antenne de réception (3) étant contraint(e) audit élément de support (10), le dispositif comprenant en outre des premiers moyens de rotation (5) associés audit élément de support (10) et configurés pour déplacer ledit élément de support (10) en rotation, déterminant une rotation correspondante du lanceur (2) ou de l'antenne de réception (3) dans une pluralité de positions autour du siège d'inspection (4a).

8. Dispositif selon l'une quelconque des revendications précédentes, comprenant des seconds moyens de rotation (6) associés au guide d'ondes (4) et configurés pour déplacer ledit guide d'ondes (4) en rotation dans une pluralité de positions autour d'un centre géométrique du siège d'inspection (4a).

9. Dispositif selon l'une quelconque des revendications précédentes 1-8, comprenant des seconds moyens de rotation (6) associés au matériau (M) à inspecter et configurés pour déplacer ledit matériau (M) en rotation dans une pluralité de positions coaxialement par rapport à un centre géométrique du siège d'inspection (4a).

10. Dispositif selon l'une quelconque des revendications précédentes, comprenant un élément de mouvement (7) configuré pour modifier une distance relative entre le guide d'ondes (4) et le matériau (M) à inspecter, déterminant une insertion plus ou moins grande du matériau (M) à inspecter à l'intérieur du siège d'inspection (4).

11. Dispositif selon l'une quelconque des revendications précédentes, dans lequel le guide d'ondes (4) comprend, dans au moins une portion d'au moins un bord externe du guide d'ondes (4), un revêtement constitué d'un matériau absorbant (M) adapté pour absorber au moins partiellement le champ électromagnétique émis dans le guide d'ondes.

12. Dispositif selon l'une quelconque des revendications précédentes, dans lequel le guide d'ondes (4) est configuré pour propager le champ électromagnétique d'une pluralité de modes de propagation vers le matériau.

13. Appareil de mesure par micro-ondes, comprenant :
- un dispositif de mesure (1) selon une ou plusieurs des revendications précédentes ;
- une unité de traitement configurée pour :
• traiter une pluralité de valeurs indiquant l'amplitude et la phase du champ électromagnétique réfléchi par le matériau dans une pluralité de positions relatives différentes entre le matériau et l'antenne de réception ;
• générer, en fonction dudit traitement, une image représentative d'une carte d'homogénéité diélectrique du matériau et un signal représentatif de la présence ou de l'absence d'une non-intégrité du matériau.
